# EUROPEAN PATENT SPECIFICATION

(11) **EP 2 117 648 B1**
(45) Date of publication and mention of the grant of the patent: **26.08.2015**
(21) Application number: 08719540.0
(22) Date of filing: 03.03.2008
(51) Int. Cl.: A61N 5/06

(54) **LIGHTING SYSTEM FOR ENERGY STIMULATION**
BELEUCHTUNGSSYSTEM ZUR ENERGIESTIMULIERUNG
SYSTÈME D'ÉCLAIRAGE POUR UNE STIMULATION ÉNERGÉTIQUE

(30) Priority: 09.03.2007 EP 07103861
(43) Date of publication of application: 18.11.2009
(73) Proprietor: Koninklijke Philips N.V., 5656 AE Eindhoven (NL)
(72) Inventor: VAN WOUDENBERG, Roel, 5656 AE Eindhoven (NL); BOLECH, Mark, 5656 AE Eindhoven (NL); GROENESTEIN, Ronald, P., 5656 AE Eindhoven (NL); HUIJBRECHTS, Paulus, A., H., J., 5656 AE Eindhoven (NL); HUIJGEN, Gerrit, J., T., 5656 AE Eindhoven (NL); VAN KAATHOVEN, Dirk, J., 5656 AE Eindhoven (NL)
(74) Representative: Bekkers, Joost J.J.
(86) International application number: PCT/IB2008/050764
(87) International publication number: WO 2008/110959

(56) References cited:
- EP-A- 1 285 676
- DE-A1-102005 027 262
- JP-A- 2003 288 995
- JP-A- 2005 063 687
- US-A- 5 824 024
- US-A1- 2003 231 495

## Description

### FIELD OF THE INVENTION

The present invention relates to a lighting system and a method for stimulating a user's energy.

### BACKGROUND OF THE INVENTION

In recent years, several lighting systems have been developed for various purposes, such as lighting systems enabling users to set color temperatures in a variable manner in accordance with time and other situations so as to provide a physiological and psychological effect such as stimulation and relaxation of blood circulation. Other types of lighting systems that have been developed in recent years are systems coupled to shower baths, which systems turn on automatically when a person takes a shower.

One important aspect that has not been taken into account in prior-art lighting systems relates to stimulating a user's energy, e.g. for enhancing his circadian rhythm.

JP 2005 063687 A discloses a system similar to the one defined in claim 1 without teaching tuning as claimed.

### OBJECT AND SUMMARY OF THE INVENTION

It is an object of the present invention to realize the above-mentioned aspect by providing a lighting system and a method for stimulating a user's energy.

According to one aspect, the present invention relates to a lighting system for stimulating a user's energy, the lighting system comprising:
- at least one light source, and
- a control unit for controlling the light emitted from the at least one light source,
wherein the controlling comprises the steps of instructing the at least one light source to emit bluish wake-up light and tuning the bluish light towards a daytime-like white light.

A lighting system is thus provided which is especially suitable to enhance a user's circadian rhythm, i.e. it improves his physical and/or mental state in that the lighting system animates the early-morning light when the sun is low and the light appears to be bluish and thus prevents the user from getting a "light-shock" while being half asleep until the sun is higher and the light is white or whitish, thus providing a natural light effect. Such an "animation" can be essential for many users, e.g. those who have problems rising early or have a profession demanding irregular hours of sleep. The users may also be children having difficulties with rising early, or people suffering from depressions.

In one embodiment, the bluish wake-up light has a wavelength range of 390nm-470nm, e.g. 410nm-450nm, 415-425nm, or 419nm-421nm.

Recent research studies have shown that bluish light within this wavelength range, preferably around 420nm, influences the melatonin suppression. For the circadian rhythm after e.g. a period of night-time shifts or periods in a different time zone, periodic exposure at this wavelength range, particularly at 420nm, can be extremely helpful.

In one embodiment, the at least one light source is selected from:
- Light-Emitting Diodes (LED),
- fluorescent lighting,
- incandescent lamps,
- halogen lamps,
- High-Intensity Discharge (HID) lamps, or
- combinations of one or more of the above light sources.

It is thus possible to cover a wide wavelength range by implementing one or more of these light sources.

In one embodiment, the bluish light is tuned towards the daytime-like white light over a given period of time manually selected by the user.

A user-friendly way is thus provided, which allows the user to personally select the most suitable period of time.

In one embodiment, the system further comprises a sensor adapted to be coupled to the control unit, wherein the tuning of the bluish light towards the daytime-like white light is initiated in response to a signal indicating the user's presence.

If the lighting system is placed in, for example, a shower booth, the sensor may be adapted to react to the user's movement or body heat in that the lighting system or cycle is turned on, and is also turned off when the user leaves the booth.

In one embodiment, the controlling further comprises the steps of emitting daytime-like light and tuning the daytime-like light to dusk-like/sunset-like light.

The lighting system can thus be implemented to help the user to relax, which may be particularly beneficial to those who work in shifts and come home in the middle of the day.

In one embodiment, the system further comprises an activation unit comprising a timer, the timer being adapted to receive an input of a start time and the activation unit being adapted to activate, in response to the received start time, the control unit to start the emission of bluish light.

The lighting system can thus be implemented as a kind of "alarm clock" enabling the user to set the start time for the bluish light.

In one embodiment, the timer is further adapted to receive an input of an end time, the difference between the received start time and the received end time indicating the maximum period of time for tuning the bluish light towards the daytime-like white light.

The user can select the maximum period of time for tuning the light from bluish towards daytime like-white, and thus adapt the system to his needs.

According to another aspect, the present invention relates to use of the lighting system in a humid environment. In one embodiment, the humid environment is a shower booth.

According to yet another aspect, the present invention relates to a method of stimulating a user's energy, the method comprising the steps of:
- providing at least one light source, and
- controlling the light emitted from the at least one light source,
wherein the controlling comprises the steps of instructing the at least one light source to emit bluish wake-up light and tuning the bluish light towards a daytime-like white light.

In one embodiment, shortly before or immediately after the daytime-like white light has been reached, the light is made warmer by increasing the red-component intensity or reducing the blue component, or by a combination thereof.

In one embodiment, the bluish light is tuned towards a daytime-like light in a continuous way.

Each aspect of the present invention may be combined with any one of the other aspects. These and other aspects of the invention are apparent from and will be elucidated with reference to the embodiments described hereinafter.

### BRIEF DESCRIPTION OF THE DRAWINGS

In the drawings,
Figure 1 shows a lighting system according to the present invention for stimulating a user's energy,
Figure 2 shows one example of implementation of the lighting system, and
Figure 3 shows a method according to the present invention for stimulating a user's energy.

### DESCRIPTION OF EMBODIMENTS

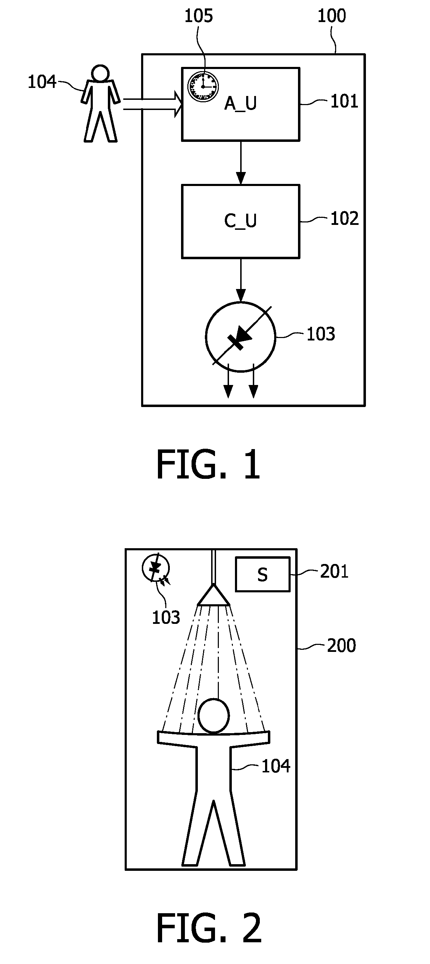

Figure 1 shows a lighting system 100 according to the present invention for stimulating a user's (104) energy, the system comprising at least one light source 103 and a control unit (C_U) 102 for controlling the light emitted from the at least one light source. The control unit (C_U) 102, which may be e.g. a single switch or any kind of more sophisticated interface, is adapted to control the at least one light source 103 by instructing it to emit bluish wake-up light and tune the bluish light towards daytime-like white light, which may be done over a given period of time selected by the user 104. In one embodiment, the bluish wake-up light has a wavelength range of 390nm-470nm, e.g. 410nm-450nm, 415-425nm, 419nm-421nm, or more precisely 420nm. Use of the wavelength within this range, preferably around 420nm, has the advantage that exposure to UV light (below 400nm) is prevented, and that it is 100 to 200 times more effective than incandescent light. Moreover, it has an emotional and physiological effect on users. A great many studies have been made on the effect of light levels and light colors on melatonin suppression. Melatonin is also known as the sleepiness hormone and the human's indicator for the time of day, or rather the phase of the day. Periodical exposure to a high brightness level is helpful for the circadian rhythm after e.g. a period of night-time shifts or a period in a different time zone. Research has shown that 1 minute of exposure to 2 klx (white light) has a clear waking effect (Glickman, G. et al., J. Biol. Rhythms, 18, 71-79 (2003); Zeitzer, J. et al. J. Physiol. 526, 695 -702 (2000)) with a maximum sensitivity to the circadian rhythm at around 420nm. As people when taking a shower are generally undressed and have a large skin area exposed to the light, exposure to UV light is prevented by using the wavelength preferably in the range around 420nm . For reasons of health, it may be preferred to use LEDs rather than e.g. fluorescent gas discharge lamps, which always have a UV component. Moreover, as the voltage across the LEDs is only as low as 2.0-4.0 volts per LED, a LED-based system can easily be made to use safe voltages, e.g. of 24V maximum, which renders them intrinsically safe for use in wet environments such as shower booths. Other light sources may of course be implemented and even required for tuning the bluish light towards the daytime-like light, such as halogen lighting systems, halogen spots, diffuse halogen lamps, HID, fluorescent lighting, incandescent lamps and halogen lamps, high-intensity discharge lamps and the like.

In one embodiment, the control operation further comprises the steps of instructing the at least one light source 103 to emit daytime-like light and transforming the daytime-like light to dusk-like/sunset-like light, e.g. over a given period of time. The lighting system can thus be implemented to enhance the user's circadian rhythm.

In one embodiment, the system further comprises an activation unit (A_U) 101 comprising a timer 105, the timer being adapted to receive an input of a start time and the activation unit being adapted to activate, in response to the received start time, the control unit (C_U) 102 to start the emission of the bluish light. For example, the user 104 may set the timer to a time in the morning when he wants the lighting system 100 to start the wake-up. Accordingly, the lighting system 100 can be implemented as a kind of "alarm clock" enabling the user 104 to set the start time for the bluish light. The timer 105 may of course also be adapted to receive an input of an end time, in which the difference between the received start time and the received end time indicates the maximum period of time for tuning the bluish light towards the daytime-like white light. Accordingly, when the user 104 sets the start time at 07:00 a.m. and the end time at 07:15 a.m., the lighting system 100 starts emitting bluish light at 07:00 a.m. and tunes it towards daytime-like white light within maximally 15 minutes, i.e. the tuning period is max. 15minutes.

Figure 2 shows an example of the lighting system 100, placed in a shower booth 200, as used by many people as part of their daily routine. Incorporation of this lighting system 100 in a shower booth 200 provides an optimal wake-up system giving the user 104 a more refreshed and awakened feeling. For example, at the beginning of the shower, the user 104 will experience a bluish light level, which prevents him from getting a light shock when entering the shower half asleep. Then, the blue light level is increased to get the optimal wake-up effect of the blue light. Shortly before the end or immediately after the shower period, the white light is made warmer by increasing the red-component intensity and/or reducing the blue component. Another example is a sensor (201) placed inside the shower booth and reacting to the user's movement or body heat in that the sensor turns on the lighting system or cycle and also turns it off when the user leaves the booth.

In one embodiment, the lighting system 100 is coupled to the shower system in such a way that the lighting system 100 starts emitting the bluish light when the water tap is turned on. The system may also be self-adaptive to the user's habits, e.g. when the user 104 usually stays in the shower booth for 15 minutes, the period of time for tuning the light from bluish light towards daytime-like white light will also be 15minutes, or even slightly shorter.

Since photoreceptors have their highest concentration in the lower half of the human eye, the light source 103 is preferably placed above the user, at angles of 20° to 70° with respect to the horizon. As the person taking a shower will generally look a bit downwards so as to prevent water entering his eyes, the light will enter his eyes at an angle of between approx. 40° and close to 90°.

The lighting system may of course be suitable for home use, e.g. in living rooms, above a bath tub, or in hotel rooms, etc.

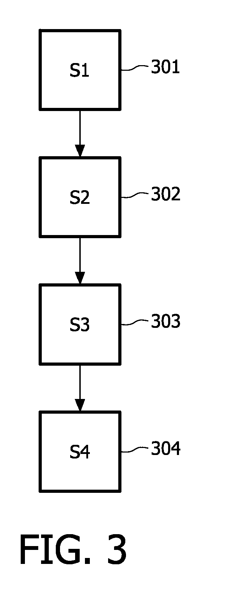

Figure 3 shows a method of stimulating a user's energy according to the present invention, wherein at least one light source is provided (S3) 303 and a control unit for controlling (S4) 304 the light emitted from the at least one light source, and wherein the control operation comprises the steps of instructing the at least one light source to emit bluish wake-up light and tuning the bluish light towards a daytime-like white light.

In one embodiment, the invention further comprises the steps of receiving a first input (S1) 301 from the user indicating a time to start emitting the bluish wake-up light, and receiving a second input (S2) 302 from the user, indicating the end time of the wake-up or the period of time.

Certain specific details of the disclosed embodiment are set forth for purposes of explanation rather than limitation so as to provide a clear and thorough understanding of the present invention. However, it should be understood by those skilled in the art that the present invention may be practiced in other embodiments that do not conform exactly to the details set forth herein, without departing from the scope of this disclosure. Furthermore, in this context, and for purposes of brevity and clarity, detailed descriptions of well-known apparatuses, circuits and methodologies have been omitted so as to avoid unnecessary detail and possible confusion.

Reference signs in the claims have only been included for clarity reasons and should not be construed as limiting the scope of the claims.

## Claims

1. A lighting system (100) for stimulating a user's (104) energy, the lighting system comprising:
- at least one light source (103), and
- a control unit (102) for controlling the light emitted from the at least one light source (103) and being configured to instruct the at least one light source (103) to emit bluish wake-up light and tune the bluish light towards a daytime-like white light.

2. A lighting system according to claim 1, wherein the bluish wake-up light has a wavelength range of 390nm-470nm, preferably 410nm-450nm, 415-425nm, or 419nm-421nm.

3. A lighting system according to claim 1, wherein the at least one light source (103) is selected from:
- Light-Emitting Diodes (LED),
- fluorescent lighting,
- incandescent lamps,
- halogen lamps,
- High-Intensity Discharge (HID) lamps, or
- combinations of one or more of the above light sources.

4. A lighting system according to claim 1, wherein the bluish light is tuned towards the daytime-like white light over a given period of time manually selected by the user (104).

5. A lighting system according to claim 1, further comprising a sensor (201) adapted to be coupled to the control unit (102), wherein the tuning of the bluish light towards the daytime-like white light is initiated in response to a signal indicating the user's (104) presence.

6. A lighting system according to claim 1, wherein the controlling further comprises the steps of emitting daytime-like light and tuning the daytime-like light to dusk-like/sunset-like light.

7. A lighting system according to claim 1, further comprising an activation unit (101) comprising a timer (105), the timer being adapted to receive an input of a start time and the activation unit being adapted to activate, in response to the received start time, the control unit to start the emission of bluish light.

8. A lighting system according to claim 7, wherein the timer (105) is further adapted to receive an input of an end time, the difference between the received start time and the received end time indicating the maximum period of time for tuning the bluish light towards the daytime-like white light.

9. Use of a lighting system as claimed in claim 1 in a humid environment.

10. Use of a lighting system according to claim 9, wherein the humid environment is a shower booth (200).

11. A method of stimulating a user's energy, the method comprising the steps of:
- providing (303) at least one light source, and
- controlling (304) the light emitted from the at least one light source,
wherein the controlling comprises the steps of instructing the at least one light source to emit bluish wake-up light and tuning the bluish light towards a daytime-like white light.

12. A method according to claim 11, wherein, shortly before or immediately after the daytime-like white light has been reached, the light is made warmer by increasing the red-component intensity or reducing the blue component, or by a combination thereof.

13. A method according to claim 11, wherein the bluish light is tuned towards a daytime-like light in a continuous way.

## Patentansprüche

1. Beleuchtungssystem (100) zur Stimulierung der Energie eines Benutzers (104), wobei das Beleuchtungssystem umfasst:
- mindestens eine Lichtquelle (103) sowie
- eine Steuereinheit (102) zur Steuerung des von der mindestens einen Lichtquelle (103) emittierten Lichts;
und so eingerichtet ist, dass es die mindestens eine Lichtquelle (103) anweist, bläuliches Wecklicht zu emittieren und das bläuliche Licht auf ein tageszeitähnliches weißes Licht abzustimmen.

2. Beleuchtungssystem nach Anspruch 1, wobei das bläuliche Wecklicht einen Wellenlängenbereich von 390nm-470nm, vorzugsweise 410nm-450nm, 415nm-425nm oder 419nm-421nm, aufweist.

3. Beleuchtungssystem nach Anspruch 1, wobei die mindestens eine Lichtquelle (103) ausgewählt wird aus:
- Licht emittierenden Dioden (LED);
- Fluoreszenzbeleuchtung;
- Glühlampen;
- Halogenlampen,
- Hochleistungsentladungs-(HID-) Lampen oder
- Kombinationen aus einer oder mehreren der obigen Lichtquellen.

4. Beleuchtungssystem nach Anspruch 1, wobei das bläuliche Licht über eine von dem Benutzer (104) manuell ausgewählte vorgegebene Zeitperiode auf das tageszeitähnliche weiße Licht abgestimmt wird.

5. Beleuchtungssystem nach Anspruch 1, das weiterhin einen Sensor (201) umfasst, der so eingerichtet ist, dass er mit der Steuereinheit (102) gekoppelt ist, wobei das Abstimmen des bläulichen Lichts auf das tageszeitähnliche weiße Licht in Reaktion auf ein die Anwesenheit eines Benutzers (104) meldendes Signal eingeleitet wird.

6. Beleuchtungssystem nach Anspruch 1, wobei die Steuerung weiterhin die Schritte des Emittierens von tageszeitähnlichem Licht und Abstimmens des tageszeitähnlichen Lichts auf dämmerungsähnliches/abendlichtähnliches Licht umfasst.

7. Beleuchtungssystem nach Anspruch 1, das weiterhin eine Aktivierungseinheit (101) mit einem Timer (105) umfasst, wobei der Timer so eingerichtet ist, dass er eine Eingabe einer Startzeit empfängt, und die Aktivierungseinheit so eingerichtet ist, dass sie die Steuereinheit in Reaktion auf die empfangene Startzeit aktiviert, um die Emission von bläulichem Licht zu starten.

8. Beleuchtungssystem nach Anspruch 7, wobei der Timer (105) weiterhin so eingerichtet ist, dass er eine Eingabe einer Endzeit empfängt, wobei die Differenz zwischen der empfangenen Startzeit und der empfangenen Endzeit die maximale Zeitperiode zur Abstimmung des bläulichen Lichts auf das tageszeitähnliche weiße Licht anzeigt.

9. Verwendung eines Beleuchtungssystems nach Anspruch 1 in einer feuchten Umgebung.

10. Verwendung eines Beleuchtungssystems nach Anspruch 9, wobei es sich bei der feuchten Umgebung um eine Duschkabine (200) handelt.

11. Verfahren zur Stimulierung der Energie eines Benutzers, wobei das Verfahren die folgenden Schritte umfasst, wonach:
- mindestens eine Lichtquelle vorgesehen wird (303); und
- das von der mindestens einen Lichtquelle emittierte Licht gesteuert wird (304),
wobei die Steuerung die Schritte des Anweisens der mindestens einen Lichtquelle, bläuliches Wecklicht zu emittieren und das bläuliche Licht auf ein tageszeitähnliches weißes Licht abzustimmen, umfasst.

12. Verfahren nach Anspruch 11, wobei kurz vor oder unmittelbar nach dem Erreichen des tageszeitähnlichen weißen Lichts das Licht durch Erhöhen der Intensität der Rotkomponente oder Reduzieren der Blaukomponente oder durch eine Kombination derselben wärmer wird.

13. Verfahren nach Anspruch 11, wobei das bläuliche Licht in einer kontinuierlichen Weise auf ein tageszeitähnliches Licht abgestimmt wird.

## Revendications

1. Système d'éclairage (100) pour stimuler l'énergie d'un utilisateur (104), le système d'éclairage comprenant :
- au moins une source lumineuse (103), et
- une unité de commande (102) pour commander la lumière émise à partir de l'au moins une source lumineuse (103),
et étant configurée pour ordonner à l'au moins une source lumineuse (103) d'émettre une lumière de réveil bleutée et d'ajuster la lumière bleutée vers une lumière blanche de jour.

2. Système d'éclairage selon la revendication 1, dans lequel la lumière de réveil bleutée a une plage de longueur d'onde de 390 nm à 470 nm, de préférence de 410 nm à 450 nm, de 415 nm à 425 nm ou de 419 nm à 421 nm.

3. Système d'éclairage selon la revendication 1, dans lequel l'au moins une source lumineuse (103) est sélectionnée parmi :
- des diodes électroluminescentes (LED),
- un éclairage fluorescent,
- des lampes incandescentes,
- des lampes halogènes,
- des lampes à décharge de haute intensité (HID), ou
- des combinaisons d'une ou plusieurs des sources lumineuses susmentionnées.

4. Système d'éclairage selon la revendication 1, dans lequel la lumière bleutée est ajustée vers la lumière blanche de jour au cours d'une période de temps donnée manuellement sélectionnée par l'utilisateur (104).

5. Système d'éclairage selon la revendication 1, comprenant en outre un capteur (201) apte à être couplé à l'unité de commande (102), dans lequel l'ajustement de la lumière bleutée vers la lumière blanche de jour est initié en réponse à un signal indiquant la présence de l'utilisateur (104).

6. Système d'éclairage selon la revendication 1, dans lequel la commande comprend en outre les étapes de l'émission d'une lumière de jour et l'ajustement de la lumière de jour à une lumière de crépuscule/de coucher de soleil.

7. Système d'éclairage selon la revendication 1, comprenant en outre une unité d'activation (101) comprenant une minuterie (105), la minuterie étant apte à recevoir une entrée d'une heure de début et l'unité d'activation étant apte à activer, en réponse à l'heure de début reçue, l'unité de commande pour commencer l'émission de la lumière bleutée.

8. Système d'éclairage selon la revendication 7, dans lequel la minuterie (105) est en outre apte à recevoir une entrée d'une heure de fin, la différence entre l'heure de début reçue et l'heure de fin reçue indiquant la période de temps maximale pour ajuster la lumière bleutée vers la lumière blanche de jour.

9. Utilisation d'un système d'éclairage selon la revendication 1 dans un environnement humide.

10. Utilisation d'un système d'éclairage selon la revendication 9, dans lequel l'environnement humide est une cabine de douche (200).

11. Procédé de stimulation de l'énergie d'un utilisateur, le procédé comprenant les étapes de :
- la fourniture (303) d'au moins une source lumineuse, et
- la commande (304) de la lumière émise à partir de l'au moins une source lumineuse,
dans lequel la commande comprend les étapes de l'instruction à l'au moins une source lumineuse d'émettre une lumière de réveil bleutée et de l'ajustement de la lumière bleutée vers une lumière blanche de jour.

12. Procédé selon la revendication 11, dans lequel, peu de temps avant ou immédiatement après que la lumière blanche de jour est atteinte, la lumière est rendue plus chaude par l'augmentation de l'intensité de la composante rouge ou par la réduction de la composante bleue, ou par une combinaison de celles-ci.

13. Procédé selon la revendication 11, dans lequel la lumière bleutée est ajustée vers une lumière de jour d'une manière continue.
